# EUROPÄISCHE PATENTSCHRIFT

(11) **EP 1 487 422 B1**
(45) Veröffentlichungstag und Bekanntmachung des Hinweises auf die Patenterteilung: **01.08.2007**
(21) Anmeldenummer: 03711965.8
(22) Anmeldetag: 11.03.2003
(51) Int. Cl.: A61K 9/50

(54) **FILMÜBERZÜGE MIT SEHR KONTROLLIERTER FREISETZUNG UND HOHER STABILITÄT**
FILM COATINGS WITH A HIGHLY CONTROLLED RELEASE AND A HIGH STABILITY
FILMS DE REVETEMENT A LIBERATION TRES CONTROLEE ET A GRANDE STABILITE

(30) Priorität: 14.03.2002 DE 10211289
(43) Veröffentlichungstag der Anmeldung: 22.12.2004
(73) Patentinhaber: BASF Aktiengesellschaft, 67056 Ludwigshafen (DE)
(72) Erfinder: KOLTER, Karl, 67117 Limburgerhof (DE); BODMEIER, Roland, 12169 Berlin (DE); DASHEVSKIY, Andriy, 12249 Berlin (DE)
(86) Internationale Anmeldenummer: PCT/EP2003/002455
(87) Internationale Veröffentlichungsnummer: WO 2003/075898

(56) Entgegenhaltungen:
- EP-A- 0 342 522
- EP-A- 0 587 182
- EP-A- 1 110 544
- WO-A-01/80829
- WO-A-97/02020

## Beschreibung

Die vorliegende Erfindung betrifft die Kombination von Polyvinylacetat mit wasserunlöslichen, säure- oder alkaliunlöslichen Polymeren zur Herstellung von Filmüberzügen für Darreichungsformen mit kontrollierter Wirkstofffreisetzung sowie Verfahren zur Herstellung derselben. Durch diese Kombinationen können die controlled-release-Eigenschaften gezielt eingestellt werden und es resultieren Filme von ausgezeichneter mechanischer Stabilität und Lagerstabilität. Insbesondere weisen die erfindungsgemäßen Darreichungsformen eine pH-unabhängige Wirkstofffreisetzung auf.

An Darreichungsformen mit verzögerter Wirkstofffreisetzung werden sehr hohe Anforderungen gestellt, denn aufgrund der im Vergleich zu schnellfreisetzenden Formen höheren Wirkstoffdosis, liegt das Gefährdungspotential bei plötzlicher schneller Wirkstofffreisetzung sehr viel höher. Werden in kurzer Zeit große Wirkstoffmengen freigesetzt und resorbiert, können Überdosierungen mit entsprechenden Nebenwirkungen bis hin zu Intoxikationen auftreten. Diese Problematik stellt sich besonders bei Arzneistoffen mit verhältnismäßig geringer therapeutischer Breite. Veränderungen der Wirkstofffreisetzung können sehr verschiedene Ursachen haben. So können sich Risse und Poren im Filmüberzug bilden, einerseits bedingt durch Quellung des Kernes, andererseits aber auch durch Spannungen im Überzug. Inhomogenitäten im Film fördern Veränderungen der Permeabilität sehr stark. Diese Inhomogenitäten sind zurückzuführen auf größere oder agglomerierte Pulverteilchen, die vom Abrieb des Kernes herrühren können oder durch zu schnelle Trocknung der Sprühsuspension entstehen können. Auch eine ungleichmäßige Versprühung bzw. die Separierung/Agglomeration von Bestandteilen der Sprühdispersion kann Inhomogenität des Filmüberzuges bewirken. Häufig haften auch die Filmüberzüge sehr schlecht auf dem Substrat und können sich ablösen.

Darüber hinaus ist verständlich, dass ein Zusammenkleben der Kerne während des Sprühprozesses gefolgt von einem Auseinanderreißen durch die wirbelnde bzw. rotierende Bewegung des Kernbettes zu Schäden im Filmüberzug führt, die auch durch weitere Auftragschichten nicht vollständig beseitigt werden können. Dieses Verhalten findet sich besonders bei klebrigen Polymeren wie Ethylacrylat-Methylmethacrylat-Copolymer (Eudragit NE 30 D, Kollicoat EMM 30 D). Überzogene Darreichungsformen müssen so stabil sein, dass sie sowohl den mechanischen Stress während des Coatingprozesses aushalten, bei dem der Film noch recht feucht ist, wie auch den mechanische Stress bei der weiteren Verarbeitung z.B. bei Abfüllen in Kapseln, bei dem der Film völlig abgetrocknet ist. Es darf zu keinen mechanischen Schäden des Filmes kommen, da diese unweigerlich eine starke Beschleunigung der Wirkstofffreisetzung zur Folge hätten.

Besondere Ansprüche sind an überzogene Formlinge zu stellen, die anschließend zu Tabletten verpreßt werden sollen, die wiederum nach oraler Applikation im Magen in die ursprünglichen gecoateten Formlinge zerfallen. Durch den Verpressungsschritt wird das Abfüllen in Kapseln ersetzt und damit ein teurer Verfahrensschritt sowie die Verwendung tierischen Materials (Gelatine) vermieden. Die Wirkstofffreisetzung soll sich durch die Verpressung nicht verändern, d.h. der Filmüberzug muß plastisch verformbar sein und darf keinen Schaden nehmen.

Viele controlled release Polymere wie Ethylcellulose oder Ammoniummethacrylat-Copolymer (Ethylacrylat-Methylmethacrylat-Trimethylammoniumethylmethacrylatchlorid) sind sehr spröde, verfilmen schlecht und benötigen große Mengen an Weichmacher. Diese große Mengen an Weichmacher sind im Film relativ frei verfügbar, migrieren im Film, migrieren aus dem Film in den Kern bzw. gehen Wechselwirkungen mit weiteren Bestandteilen der Darreichungsform ein. Daraus resultiert dann auch-eine Veränderung der Wirkstofffreisetzung. Die Weichmacherkonzentration in Filmüberzügen sollte aus den genannten Gründen so niedrig wie möglich sein. Bislang gibt es keine zufriedenstellenden Lösungen für dieses Problem.

Zahlreiche Arzneistoffe sind pH-abhängig löslich und daher zeigen herkömmliche Retardformen eine pH-abhängige Freisetzung. Arzneistoffe stellen in der Regel schwache Basen oder schwache Säuren bzw. Salze derselben dar. Ein Hydrochlorid einer schwachen Base weist in der Regel eine höhere Löslichkeit bei saurem pH-Wert auf, wohingegen eine Säure oder das Alkalisalz einer Säure in Neutralbereich bzw. im Alkalischen besser löslich ist. Bei dem pH-Wert, bei dem die Löslichkeit des Wirkstoffes am größten ist, ist auch in der Regel die Wirkstofffreisetzung am schnellsten, da das Konzentrationsgefälle von der Filminnenseite zur -außenseite dann am größten ist. Zur Lösung dieses Problems gab es bisher verschiedene Versuche, die allerdings allesamt bislang nicht zufriedenstellend sind und teilweise gravierende Nachteile aufweisen.

In dem EP 0438249 wird eine Naproxenformulierung beschrieben, die mit mehreren Schichten einer Kombination aus einem wasserunlöslichen und einem wasserlöslichen Polymer gecoatet ist. Die beanspruchten wasserlöslichen Polymere zeigen keine pH-Abhängigkeit der Löslichkeit und sind daher nicht in der Lage die Diffusion entsprechend der Löslichkeit des Wirkstoffes zu steuern. Das Aufbringen dieser Mehrschichtenmembran ist zudem sehr aufwendig und mit dem Gebrauch von organischen Lösungsmitteln verbunden. Als weitere Besonderheit ist in den Kern neben dem Wirkstoff eine größere Menge einer organischen Säure eingearbeitet, wodurch die Wirkstoffkonzentration entsprechend reduziert wird. Die gecoateten Teilchen werden dann mit nicht gecoateten zu Tabletten verpresst. Insgesamt ist dieses Herstellverfahren sehr umständlich und mit hohen Kosten verbunden.

Das US Patent 5175003 beschreibt controlled release Pellets mit säurelöslichen Arzneistoffen, wobei der Überzug aus einer. Kombination eines magensaftresistenten Polymers mit einem wasserlöslichen Polymer besteht. Durch das magensaftresistente Polymer wird die Freisetzung im Sauren stark verzögert. Im Neutralbereich bzw. im Alkalischen zerfällt der Überzug oder löst sich ganz auf. Dadurch ist in diesem Bereich keine gezielte Steuerung der Freisetzung durch den Überzug mehr möglich. Hinzu kommt, dass sich bei Patienten, die wenig Magensäure haben bzw. entsprechende Medikamente zur Abpufferung der Magensäure nehmen und folglich einen höheren pH-Wert im Magen aufweisen, der Überzug schnell auflöst und somit keine nennenswerte Retardierung mehr vorhanden ist.

Die EP 0287536 beschreibt pharmazeutische, gecoatete Chinidinformulierungen mit retardierter Freisetzung, die dadurch gekennzeichnet sind, dass sie ein bestimmtes Freisetzungsprofil aufweisen. Der Überzug besteht aus einer Kombination von Hydroxypropylmethylcellulosephthalat (70 bis 35 %) mit unlöslichen Polymeren, wie Ethylcellulose oder Ethylacrylat-methylmethacrylattrimethylammoniumethylmethacrylat-Copolymer (30 bis 65 %) . Polyvinylacetat ist in keinster Weise erwähnt. Der Überzug muss zudem aus organischer Lösung aufgebracht werden, die die bekannten zahlreichen Nachteile wie Explosionsgefahr, Toxizität, hohe Kosten etc. mit sich bringt. Die beschriebenen Überzüge sind ausgesprochen spröde und widerstehen mechanischer Belastung nicht.

In der PCT 99/01129 wird ein Prozess zur Herstellung einer controlled release Form beansprucht, die geeignet ist, einen Dihydropyridin-Calciumantagonisten in flüssiger Form zu applizieren. Dabei werden Mikrogranalien mit mehreren Schichten verschiedener Polymere überzogen. Bei der 1. Schicht handelt es sich um eine lipophile pH-unempfindliche Schicht, die weiteren bestehen abwechselnd aus hydrophilen und lipophilen Bestandteilen. Es ist leicht nachzuvollziehen, dass ein solcher Aufbau sehr umständlich ist, die Applikation lange Zeit erfordert und die gesamte Herstellung und damit auch die Darreichungsform sehr teuer ist. Den Erfindern war es anscheinend nicht möglich, einen einfacheren Aufbau zu finden.

Das in der WO 01/15668 beschriebene gecoatete Arzneimittel enthält einen heterogenen Überzug aus mindestens 75 % eines wasserunlöslichen Polymers und 1 bis 25 % eines magensaftresistenten Polymers, der in der Lage ist die Freisetzung so zu steuern, dass eine Verzögerung im sauren Milieu und eine Beschleunigung im neutralen, bzw. alkalischen Milieu auftritt. Somit setzt das Coating nicht pH-unabhängig frei, sondern pH-abhängig. Das wasserunlösliche Polymer kann ein quartäres Ammoniummethacrylatpolymer, ein Acrylestercopolymer, ein Methacrylestercopolymer oder ein Celluloseether bzw. -ester sein, wobei Ethylcellulose besonders bevorzugt ist. Polyvinylacetat ist hierbei in keinster Weise erwähnt. Als magensaftresistentes Polymer können z.B. Celluloseacetatphthalat, Hydroxypropylcelluloseacetatphthalat, Polyvinylacetatphthalat oder Methacrylatcopolymer verwendet werden. Die schnelle Freisetzung im neutralen bzw. alkalischen Milieu rührt wahrscheinlich daher, dass die Mischung wie von den Erfindern beschrieben heterogen ist und im Film Bezirke mit hohem Anteil an magensaftresistentem Polymer vorliegen, die sich schnell auflösen. Aus diesen diffundiert der Wirkstoff dann sehr schnell nach außen.

Da die genannten wasserunlöslichen Polymere schlecht verfilmen bzw. klebrig sind, ist der Coatingprozess sehr aufwendig. Die beschriebenen Überzüge sind sehr spröde und widerstehen mechanischen Belastungen nicht. Schon durch die üblichen Belastungen, die beim Mischen und Abfüllen auftreten, entstehen Risse, die zu Freisetzungsveränderungen führen. Aufgrund ihrer Sprödigkeit können die beanspruchten Filmüberzüge auch Quellungsprozesse des Kernes nicht ohne Beschädigung überstehen. Solche Quellungsprozesse sind nicht zu vermeiden, da Wirkstoffe und nahezu alle pharmazeutischen Hilfsstoffe, die für die Kernherstellung verwendet werden, Wasser adsorbieren und aufnehmen, wodurch eine Volumenzunahme ausgelöst wird. Schlecht verfilmende Polymere wie es gerade die Ethylcellulose darstellt, bedingen darüber hinaus eine unzureichende Filmhomogenität, d.h. der Überzug verfilmt noch während der Lagerung nach, wodurch die Freisetzung in der Regel herabgesetzt wird. Darüber hinaus sind die hohen Konzentrationen an Weichmachern für die Stabilität von großem Nachteil, weil die Weichmacher in den Kern migrieren und auch verdunsten können, wodurch sich die Filmeigenschaften und Kerneigenschaften deutlich verändern.

Auch in US 5202128 werden pH-abhängig freisetzende Zubereitungen mit den oben genannten Nachteilen beschrieben.

Aus der EP-A 868 912 sind Überzugsmaterialien aus 10 bis 95 Gew. -% Polyvinylacetat und 5 bis 90 Gew.-% eines N-Vinylpyrrolidon-haltigen Polymers bekannt.

Aus der EP-A 1 110 544 ist die Verwendung eines Filmüberzugs, bestehend aus Polyvinylacetat und hydrophilen Zusätzen, als geschmacksmaskierendes Coating für orale Darreichungsformen bekannt, wobei die Formen im wesentlichen schnellfreisetzend sind.

Aus der WO 01/80829 sind geschmacksmaskierte Wirkstoffgranulate bekannt, die mit einem Überzug aus Polyvinylacetat, Polyvinylpyrrolidon, Ethylcellulosen und Methacrylat-Copolymeren versehen sind. Diese Überzüge sollen der Geschmacksmaskierung dienen, was bedeutet, dass die Überzüge im neutralen Bereich des Mundes die Freisetzung behindern sollen, während sie im sauren Bereich des Magens eine schnelle Freisetzung ermöglichen, also eine pH-abhängige Freisetzung ermöglichen.

Aus der EP 0587182 sind wirkstoffhaltige Granulate bekannt, die mit einer Mischung aus Polyvinylacetat und Ethylcellulose überzogen sind. Dabei handelt es sich nicht um kontrolliert pH-unabhängig freisetzende Überzüge, sondern Überzüge, die bei pH 6 die Freisetzung hindern, bei pH 3 aber für eine schnelle Freisetzung sorgen.

Aus der WO 97/02020 sind magensaftresistente Tabletten bekannt, die mit einer Polyvinylacetatschicht und einer Schicht eines säureunlöslichen Methacrylat-Copolymers überzogen sind. Solche Tabletten setzen nicht pH-unabhängig frei, da die Überzüge aus mindestens zwei Schichten bestehen, wobei die innere unter anderem aus Polyvinylacetat bestehen kann, als äussere Schicht aber ein magensaftresistenter Überzug ("enteric coating") aufgetragen wird. Dieses enterische Coating soll verhindern, dass der säurelabile Wirkstoff im Magen freigesetzt wird. Damit lösen sich die Arzneiformen im sauren pH des Magens nicht auf, sind als nicht pH-unabhängig löslich.

In der EP 0342522 sind Darreichungsformen beschrieben, die mit einer Polyvinylacetat- und einer Hydroxypropylmethylcellulosephtalat-Schicht überzogen sind, wobei das Hydroxypropylmethylcellulosephtalat als enterisches Coating dafür sorgt, dass der säurelabile Wirkstoff nicht durch die Magensäure zersetzt wird. Das enterische Coating ist im Sauren nicht löslich, jedoch im neutralen Darmmilieu löslich. Damit sind die Darreichungsformen nicht pH-unabhängig löslich.

Aufgabe der vorliegenden Erfindung war es, Filmüberzüge zu finden, die die geschilderten Nachteile vermeiden.

Demgemäß wurden mit einem Filmüberzug versehene wirkstoffhaltige Darreichungsformen mit kontrollierter pH-unabhängiger Wirkstofffreisetzung gefunden, enthaltend als Wirkstoffe Arzneistoffe, Tierarzneistoffe, Vitamine, Carotinoide, Neutraceuticals, Nahrungsergänzungsmittel oder Zusatzstoffe, Mineralstoffe oder Spurenelemente, wobei der Filmüberzug
(A) 10 - 99 Gew.-% Polyvinylacetat,
(B) 1 - 50 Gew.-% mindestens eines Polymers aus der Gruppe, bestehend aus lipophilen wasserunlöslichen Polymeren, säureunlöslichen Polymeren und alkaliunlöslichen Polymeren, und
(C) 0 - 50 Gew.-% weitere pharmazeutisch akzeptable Hilfsmittel
enthält, und die Summe der Komponenten (A), (B) und (C) 100 Gew.-% beträgt.

Durch Kombination von Polyvinylacetat mit säureunlöslichen oder alkaliunlöslichen Polymeren lassen sich die Permealibitätseigenschaften des Überzuges gezielt einstellen, so dass eine pH-unabhängige Freisetzung resultiert. Dazu wird im Falle eines bei saurem pH-wert besser löslichen Wirkstoffes ein säureunlösliches Polymer verwendet, das im Sauren die Permeabilität des Überzuges erniedrigt, aber sie im Alkalischen, wo die Löslichkeit des Arzneistoffes niedrig ist, erhöht. Bei Wirkstoffen, die im Alkalischen besser löslich sind, wird ein alkaliunlösliches Polymer eingesetzt. Löslichkeit des wirkstoffes und Permeabilität des überzuges müssen sich immer konträr verhalten. Das Verhältnis Polyvinylacetat zu säureunlöslichem bzw. alkaliunlöslichem Polymer richtet sich nach den Löslichkeitsunterschieden des wirkstoffes bei verschiedenen pH-Werten, d.h. je größer der Unterschied desto mehr säureunlösliches bzw. alkaliunlösliches Polymer muß verwendet werden. Die Obergrenze für diese Anwendung liegt ungefähr bei einem Verhältnis von 50 : 50. Die üblichen Verhältnisse liegen zwischen 99 : 1 und 70 : 30 Polyvinylacetat : säureunlösliches bzw. alkaliunlösliches Polymer. Werden zu hohe Konzentrationen an säure- bzw. alkaliunlöslichen Polymeren verwendet, ist die Integrität des Filmüberzuges nicht mehr bei allen pH-Werten gewährleistet und die Freisetzung verläuft wieder pH-abhängig.

Als säureunlösliche Polymere können Polymere ausgewählt aus der Gruppe, bestehend aus Acrylat-Methacrylsäure-Copolymere, Carboxyalkylcellulosen, Celluloseacetatphthalate, Celluloseacetatsuccinate, Celluloseacetattrimellitate, Hydroxyalkylcellulosephthalate, Hydroxyalkylcelluloseacetatsuccinate, Vinylacetatphthalate eingesetzt werden. Besonders geeignete Polymere aus diesen Klassen sind Ethylacrylat-Methacrylsäure-Copolymer, Methylmethacrylat-Methacrylsäure-Copolymer, Celluloseacetatphthalat, Celluloseacetattrimellitat, Hydroxypropylmethylcelluloseacetatphthalat, Hydroxypropylmethylcelluloseacetatsuccinat und Polyvinylacetatphthalat. Darüber hinaus kann auch Schellack verwendet werden.

Als im Neutralbereich bzw. im Alkalischen unlösliche Polymere können Anwendung finden: basische Acrylat- oder Methacrylatcopolmere wie z.B. Dimethylaminoethylmethacrylat-Methylmethacrylat-Butylmethacrylat-Terpolymer oder basische Polysaccharide wie z.B. Chitosan.

Viele der Retardpolymeren bzw. magensaftresistenten Polymeren sind sehr spröde, wodurch sich die eingangs beschriebenen Probleme ergeben. Überraschenderweise wurde nun gefunden, dass sich die Filmeigenschaften durch Zusätze von Polyvinylacetat erheblich verbessern. Die Flexibilität nimmt überproportional zu, wodurch Rissbildung und vorzeitige Freisetzung bzw. Dose-dumping vermieden werden. Der Weichmacheranteil kann verringert werden. Dadurch wird die Arzneiform stabiler bei Lagerung. Darüber hinaus kann die Schichtdicke bei verbesserten Eigenschaften reduziert werden, wodurch sich erhebliche Kosten- und Zeitersparnisse ergeben. Im Gegensatz zu reiner Ethylcellulose weist ein Film bestehend aus einer 1:1-Mischung Ethylcellulose-Polvinylacetat eine gute Flexibilität auf.

Formlinge, die mit den Polymerkombinationen mit Polyvinylacetat gecoatet sind, können zusammen mit üblichen Tablettierbestandteilen wie z.B. mikrokristalline Cellulose zu Tabletten verpresst werden, die im Magen schnell wieder in die überzogenen Formlinge zerfallen. Die Freisetzung ist unerwarteterweise gegenüber den nicht verpressten Pellets nahezu unverändert:

Die erfindungsgemäßen,Kombinationen ermöglichen überraschend in einfacher Weise die Herstellung von sehr kontrolliert freisetzenden Darreichungsformen mit hoher mechanischer Stabilität, hoher Unempfindlichkeit gegenüber äußeren Einflüssen, hoher Lagerstabilität, ausgezeichnetem Aussehen, hoher Reproduzierbarkeit und hoher Arzneimittelsicherheit. So überstehen die erfindungsgemäß gecoateten Darreichungsformen eine Friabilitätsprüfung über 60 min, wobei die Darreichungsformen 1500 mal über eine Fallhöhe von 15,5cm fallen, ohne jede Beeinflussung der Freisetzung.

Die Schwankungsbreite der Freisetzungen von verschiedenen einzelnen Formlingen bzw. Darreichungsformen wie auch die Schwankungsbreite zwischen verschiedenen Chargen ist unerwarteterweise sehr niedrig.

Die Freisetzung kann durch Auswahl und Menge der entsprechenden polymeren Kombinationspartner eingestellt werden, wobei die Kombination so gewählt wird, dass die Freisetzung unabhängig von dem gewählten Freisetzungsmedium bzw. der Magen- oder Darmflüssigkeit ist. Dadurch wird eine gleichmäßigere Arzneimittelwirkung im menschlichen Körper erzielt.

Das-Verhältnis der Kombinationspartner hängt entscheidend von den Löslichkeitsunterschieden des Arzneistoffes in der Magenflüssigkeit und der Darmflüssigkeit ab. Sind die Unterschiede groß, muss auch eine größere Menge pH-abhängig löslicher Polymere verwendet werden. Sind sie klein, genügt eine geringere Menge.

Die überzogenen Formlinge können auch Weiterverarbeitungsprozessen unterzogen werden, die mit Scher-, Zug- und Druckbelastungen einhergehen, ohne dass sich die Eigenschaften verändern. Darüber hinaus sind die erfindungsgemäßen Polymerkombinationen äußerst resistent gegenüber Umwelteinflüssen wie Feuchtigkeit und Sauerstoff.

Ein besonderer Vorzug der erfindungsgemäßen Polymerkombination ist, dass keine organischen Lösungsmittel verwendet werden müssen. Zwar können organische Lösungsmittel alleine oder auch in Kombination mit Wasser eingesetzt werden, aber auch mit rein wässrigen Zubereitungen werden die gleichen Filmeigenschaften erzielt.

Geeignete wasserunlösliche lipophile Polymere sind Ethylcellulose, Ethylacrylat-Methylmethacrylat-Copolymer, Ethylacrylat-Methylmethacrylat-Trimethylammoniumethylmethacrylatchlorid-Copolymer, Vinylacetat-Alkylacrylat oder Vinylacetat-Alkylmethacrylat-Copolymer.

Das Coating kann auch in zwei oder mehreren Schichten appliziert werden, die sich in ihrem Verhältnis Polyvinylacetat zu lipophilen, wasserunlöslichen oder säureunlöslichen oder alkaliunlöslichen Polymeren unterscheiden. Es werden somit Polyvinylacetatreiche und -arme Schichten aufgetragen.

Das Coating kann auch in zwei oder mehreren Schichten appliziert werden, wobei die eine Polyvinylacetat und die andere das lipophile, wasserunlösliche oder säureunlösliche oder alkaliunlösliche Polymer enthält. Dabei ist die Verwendung von Polyvinylacetat in der inneren Schicht zwar ebenfalls möglich, jedoch weist die Verwendung von Polyvinylacetat als äußere Schicht gewisse Vorteile auf. Aufgrund der Interaktion an der Grenzfläche der beiden Schichten haften diese sehr stark aufeinander und sind nicht mehr voneinander zu trennen.

Wenn das Coating zwei oder mehrere Schichten aufweisen soll, werden die Coatingsuspensionen zeitlich hintereinander gesprüht.

Die besondere Wechselwirkung von Polyvinylacetat mit Cellulosederivaten und Acrylat-Methacrylat-Copolymeren zeigt sich nicht nur in der Flexibilität, sondern auch in weiteren Filmeigenschaften. So wird die Rauhigkeit der Filme herabgesetzt, der Glanz erheblich verstärkt und die Klebrigkeit herabgesetzt. Die Abriebfestigkeit der Kombinationen ist höher als die der reinen Komponenten. Diese deutlichen Verbesserungen beruhen auf einem homogenen Verfilmungsprozess, der keine Schwachstellen im Filmverband erzeugt. Es gibt Hinweise darauf, dass die Verbesserung der Filmeigenschaften zum Teil auf der Ausbildung von Wasserstoffbrücken zwischen den Hydroxygruppen der Cellulose und der Esterstruktur des Polyvinylacetates beruht. In gleicher Weise können auch die Carboxylgruppen von säureunlöslichen Polymeren mit der Esterstruktur reagieren. Diese Wechselwirkung zeigt sich an einer Verschiebung der Carbonylbande des Polyvinylacetates in der Infrarotspektroskopie.

Werden zwei Dispersionen miteinander gemischt, so können Koagulationen auftreten, die aber nicht auf der Unverträglichkeit der Polymeren selbst beruhen, sondern durch Zerstörung des stabilisierenden Prinzips der Dispersionen bedingt sind. In der Regel sind Dispersionen elektrostatisch stabilisiert, d.h. die Dispersionströpfchen weisen ein negatives oder positives Zetapotential auf. Wird dieses so erniedrigt, dass es gegen Null geht, tritt sofort Koagulation auf. Dieses Phänomen zeigt sich bei der Kombination von Polyvinylacetat-Dispersion (Kollicoat^{®} SR 30 D) und Methacrylsäure/Ethylacrylat-Copolymer-Dispersion (Kollicoat MAE 30 DP).

Überraschenderweise lassen sich mit diesen als Dispersion unverträglichen Komponenten dennoch hervorragende Filme herstellen, wenn beide Dispersionen über getrennte Sprühdüsen auf Formlinge aufgesprüht wurden. Die Koagulation der feinen Dispersionsteilchen erfolgt damit direkt auf der Oberfläche der zu coatenden Formlinge. Solche Filmüberzüge zeigen keinerlei Anzeichen irgendeiner Separation von Polymerbestandteilen; sie sind glatt, glänzend, abriebfest und mechanisch außerordentlich stabil.

Ferner lässt sich die Koagulation von Dispersionen beim Mischen durch den Zusatz von oberflächenaktiven Stoffen vermeiden oder zumindest herabsetzen. Bevorzugt werden hierfür Stoffe mit einem HLB-Wert von größer 10 eingesetzt. Diese oberflächenaktiven Stoffe können ionischer oder nichtionischer Natur sein. Besonders geeignet sind Polyoxyethylensorbitanfettsäureester wie z.B. Polysorbat 80, Polyoxyethylenfettsäuren, Polyoxyethylenfettalkohole, ethoxyliertes Ricinusöl, ethoxyliertes hydriertes Ricinusöl wie z.B. Cremophor RH 40, Alkalisalze von Fettsäuren wie z.B. Natriumstearat, Alkalisalze von Alkylsulfaten bzw. -sulfonaten wie z.B. Natriumlaurylsulfat, Polyoxyethylen-Polyoxypropylen-Blockpolymere wie z.B. Lutrol F 68 oder F 127, Natriumdioctylsulfosuccinat. Übliche wirksame Konzentrationen liegen zwischen 0,2 und 20 % vorzugsweise zwischen 0,5 und 10 %.

Weiterhin können die Überzüge wasserlösliche Stoffe enthalten, die nieder- oder hochmolekular sein können. Dadurch kann die Freisetzung bei Wirkstoffen, die ein schlechtes Permeationsverhalten aufweisen, beschleunigt werden. Diese Stoffe lösen sich entweder aus dem Überzug heraus und erzeugen Poren oder sie sorgen für eine schnellere und höhere Wasseraufnahme des Filmüberzuges, wodurch die Permeation ebenfalls erhöht wird. Die pH-Unabhängigkeit der Freisetzung wird dadurch nicht beeinflusst. Beispiele für niedermolekulare wasserlösliche Stoffe sind Zucker oder Zuckeralkohole wie Lactose, Sorbit, Mannit, Xylit, Glucose, Saccharose, Salze organischer oder anorganischer Säuren wie Natriumchlorid. Kaliumacetat, Natriumphosphate, Natriumcitrate, Natriumsuccinate, Natriumtartrate oder auch Harnstoff. Als hochmolekulare Stoffe können beispielsweise eingesetzt werden: Polyvinylpyrrolidon, Vinylpyrrolidon-Vinylacetat-Copolymere, Polyvinylalkohol, Hydroxypropylcellulose, Hydroxypropylmethylcellulose, Cellulose, Maltodextrine, Galactomannane, Dextrine, Dextrane, Pektine, Xanthane, Alginate, Polydextrose, Polyethylenglykole, Polyoxyethylen-Polyoxypropylen-Blockpolymere.

Die beschriebenen Überzüge können auf die unterschiedlichsten Kerne aufgebracht werden, wie z.B. Pellets, Kristalle, Granulate, Tabletten, Drug Delivery Systeme.

Die Darreichungsformen können in den üblichen Coatinggerätschaften gecoatet werden, wie z.B. Horizontaltrommelcoater, Tauchschwertcoater, Wirbelschichtcoater, Kugelcoater, Dragierkessel. Die Zulufttemperatur kann dabei zwischen 30 und 100°C vorzugsweise zwischen 50 und 90°C betragen.

Überraschenderweise lassen sich die aufzubringenden Coatingzubereitungen sehr hoch an Feststoffen konzentrieren, wodurch der Sprühprozess sehr kurz und ökonomisch abläuft. Üblicherweise werden Feststoffkonzentrationen von 15 bis 40 % eingestellt.

Als weitere übliche Coatingbestandteile können verwendet werden:
Pigmente wie z.B. Titandioxid, Eisenoxide, verlackte Farbstoffe, wasserlösliche Farbstoffe
Suspendierhilfsstoffe
Haftverbesserer
Antiklebemittel wie z.B. Talkum, Magnesiumstearat, Stearinsäure, mikrokristalline Cellulose
Weichmacher wie z.B. Triethylcitrat, Triacetin, Propylenglykol, Diethylsebacat, Dibutylphthalat, Acetyltributylcitrat, Polyethyl-englykol, Glycerolmonostearat
Antischaummittel wie z.B. Siliconemulsionen
Tenside

Diese Stoffe dienen in aller Regel dazu, der gecoateten Form eine Farbe bzw. Aussehen zu geben sowie das Handling der Sprühsuspension und den Sprühprozess zu verbessern.

Aufgrund der guten Filmbildeigenschaften ist ein Curing d.h. eine Temperung über längere Zeit der erfindungsgemäßen Darreichungsformen nicht prinzipiell erforderlich. In bestimmten Fällen kann es allerdings die Stabilität der Wirkstofffreisetzung bei Lagerung verbessern.

Es können Wirkstoffe aus allen Anwendungsgebieten erfindungsgemäß formuliert werden. Neben Arzneimitteln lassen sich auch Tierarzneimittel, Nahrungsergänzungsmittel, Nutraceuticals, Vitamin-, Carotinoid-, Spurenelemente- und Mineralstoffpräparate auf diese weise herstellen.

Die hervorragenden Filmbildungseigenschaften der erfindungsgemäßen Zubereitungen zeigen sich auch darin, dass sehr unterschiedliche Kernformen gecoatet werden können, wie z.B. Kristalle, Granulate, Pellets, Tabletten, Extrudate, Drug Delivery Systeme.

### Beispiele

### Beispiel 1

Auf 1,08 kg Acetylsalicylsäure-Kristalle mit einer Korngröße 0,5 bis 0,8 mm wurden in einem Wirbelschichtcoater Glatt GPC Gl zwei Schichten aufgetragen. Die erste Schicht enthielt hauptsächlich Dimethylaminoethylmethacrylat-Methylmethacrylat-Butylmethacrylat-Copolymer, während die zweite Schicht hauptsächlich Polyvinylacetat als Filmbildner enthielt.

### Coatingrezeptur 1

| | |
|---|---|
| Dimethylaminoethylmethacrylat-Methylmethacrylat Butylmethacrylat-Terpolymer (Eudragit^{®} E PO) | 40,0 g |
| Polyvinylacetat-Dispersion, 30 gew.-%ig in Wasser (Kollicoat SR 30 D) | 50,0 g |
| Talkum | 5,0 g |
| Natriumlaurylsulfat | 1,0 g |
| Wasser | 250,0 g |

### Coatingrezeptur 2

| | |
|---|---|
| Polyvinylacetat-Dispersion, 30 gew.-%ig in Wasser (Kollicoat SR 30 D) | 483,3 g 483,3 g |
| Talkum | 55,0 g |
| Vinylpyrrolidon-Vinylacetat (6:4)-Copolymer (Kollidon VA 64) | 7,5 g |
| Natriumlaurylsulfat | 0,5 g |
| Wasser | 150,0 g |

Das Verhältnis Polyvinylacetat zu alkaliunlöslichem Polymer als Summe beider Schichten beträgt 8:2.

### Herstellung Sprühsuspension 1

Zur Herstellung der Sprühsuspension 1 wurden 40,0 g Eudragit E PO und 5,0 g Talkum mit 1,0 g Natriumlaurylsulfat in 250,0 g Wasser eingerührt und fein dispergiert. Dieser Suspension wurde unter Rühren 50,0 g Kollicoat SR 30 D zugesetzt.

### Herstellung Sprühsuspension 2

55,0 g Talkum, 7,5 g Kollidon VA 64 und 0,5 g Natriumlaurylsulfat wurden ebenfalls unter Rühren in 150,0 g Wasser eingerührt und diese Suspension langsam unter Rühren zu 483,3 g Kollicoat SR 30 D gegeben.

Beide Coatingsuspensionen wurden bei einer Zulufttemperatur von 55°C und einer Sprührate von 12 g/min mittels Wurstereinsatz auf die wirbelnden Kristalle appliziert. Das Coatinggewicht betrug 25 % bezogen auf die vorgelegten Acetylsalicylsäure-Kristalle.

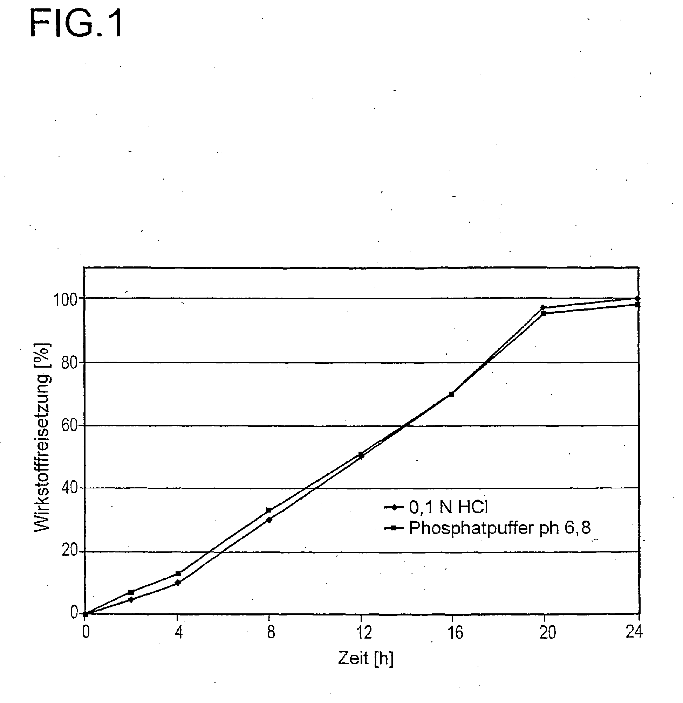

Die Freisetzung des Wirkstoffes wurde in 0,1 N HCl (künstlicher Magensaft -◆-) und in Phosphatpuffer pH 6,8 (künstlicher Darmsaft -■-) bestimmt. Die Freisetzungskurven sind in Abb. 1 dargestellt.

Acetylsalicylsäure weist eine deutlich pH-abhängige Löslichkeit auf. Im Sauren beträgt sie nur 0,45 %, aber im Neutralbereich bzw. Alkalischen liegt sie größer 25 %.

Die Freisetzung war in beiden Medien gleich schnell.

### Vergleichsbeispiel 1

In der Rezeptur von Beispiel 1 wurde Dimethylaminoethylmethacrylat-Methylmethacrylat-Butylmethacrylat-Terpolymer (Eudragit E PO) weggelassen. Der Coatingprozess wurde analog durchgeführt.

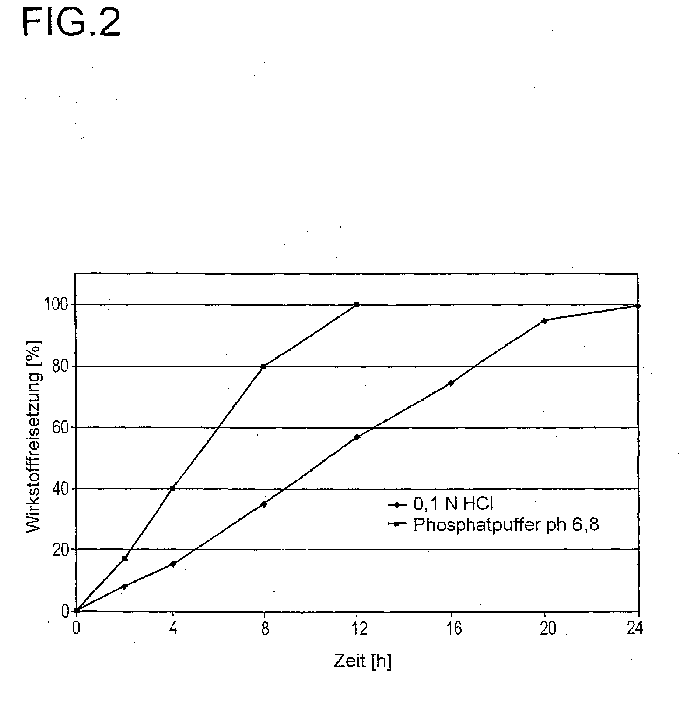

Die Freisetzung war in Phosphatpuffer pH 6,8 deutlich schneller als in 0,1 N HCl. Die Freisetzungskurven sind in Abb. 2 dargestellt.

### Beispiel 2

600 g Acetylsalicylsäure-Pellets mit einem Wirkstoffgehalt von 10 % (10 % Acetylsalicylsäure, 0,5 % Hydroxypropylmethylcellulose 5 mPas, 0,05 % Polyethylenglycol 4000, 89,45 % Saccharose), die mittels eines Drug Layering Verfahrens ausgehend von Saccharosekügelchen (Sugar spheres) hergestellt wurden, wurden in einem Huettlin Kugelcoater HKC 5 mit einem 2-schichtigen Filmüberzug gecoatet. Die erste Schicht bestand aus einem Dimethylaminoethylmethacrylat-Methylmethacrylat-Butylmethacrylat-Copolymer-Coating während die 2. Schicht Polyvinylacetat als Filmbildner aufwies.

### Coatingrezeptur 1

| | |
|---|---|
| Dimethylaminoethylmethacrylat-Methylmethacrylat Butylmethacrylat-Terpolymer (Eudragit^{®} E PO) | 30,0 g 30, 0 g |
| Dibutylsebacat | 4,5 g |
| Talkum | 30,0 g |
| Natriumlaurylsulfat | 2,1 g |
| Wasser | 316,0 g |

### Coatingrezeptur 2

| | |
|---|---|
| Polyvinylacetat-Dispersion , 30 gew.-%ig in Wasser (Kollicoat SR 30 D) | 300,0 g 300,0 g |
| Wasser | 300,0 g |

Das Verhältnis Polyvinylacetat zu alkaliunlöslichem Polymer beträgt 7,5:2,5.

### Coatingsuspension 1

Zur Herstellung der Coatingrezeptur wurden 30,0 g Eudragit E PO und 4,5 g Dibutylsebacat mit 1,0 g Natriumlaurylsulfat in 316,0 g Wasser eingerührt und fein dispergiert. 30,0 g Talkum wurden ebenfalls unter Rühren zugegeben und dispergiert.

### Coatingsuspension 2

300,0 g Polyvinylacetat-Dispersion wurden mit 300 g Wasser verdünnt.

Die Coatingdispersionen wurde bei einer Zulufttemperatur von 50°C und einer Sprührate von 6 g/min auf die wirbelnden Pellets appliziert. Die Produkttemperatur lag in beiden Fällen bei ca. 39°C.

Das Filmbildnergewicht betrug bei der 1. Schicht 5 % und bei der 2. Schicht 15 %.

Die Freisetzung des Wirkstoffes wurde in 0,1 N HCl (-◆-) und in Phosphatpuffer (-■-) pH 6,8 bestimmt. Acetylsalicylsäure weist eine deutlich pH-abhängige Löslichkeit auf. Im Sauren beträgt sie nur 0,45 %, aber im Neutralbereich bzw. Alkalischen liegt sie größer 25 %.

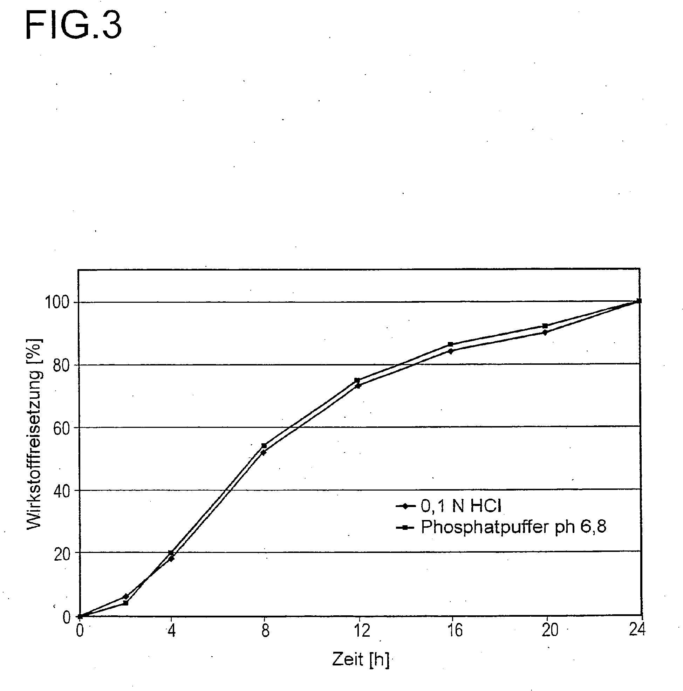

Die Freisetzung war in beiden Medien gleich schnell. Die Freisetzungskurven sind in Abb. 3 dargestellt.

### Beispiel 3

0,5 kg Verapamilpellets (bestehend aus 40 % Verapamil-HCl, 40 % mikrokristalline Cellulose, 15 % Ludipress LCE (Formulierung aus 96,5 Gew.-% Lactose und 3,5 Gew.-% Polyvinylpyrrolidon), 5 % Vinylpyrrolidon-Vinylacetat (6:4)-Copolymer) mit einer Korngröße 0,8 bis 1,5 mm wurden in einem Wirbelschichtcoater Aeromatic Stream 1 mit einem zweischichtigen Überzug gecoatet. Die erste Schicht enthielt als filmbildendes Polymer Methacrylsäure-Ethylacrylat-Copolymer, die zweite Schicht Polyvinylacetat.

### Coatingsuspension 1

| | |
|---|---|
| Methacrylsäure-Ethylacrylat-Copolymer, 30 gew. -%ig in Wasser, Dispersion (Kollicoat MAE 30 DP) | 50,0 g 50,0 g |
| Triacetin | 2,3 g |
| Polyvinylpyrrolidon K-Wert 90 | 1,7 g |
| Wasser | 46,0 g |

### Coatingsuspension 2

| | |
|---|---|
| Polyvinylacetat-Dispersion, 30 gew.-%ig in Wasser (Kollicoat SR 30 D) | 283,3 g |
| Talkum | 10,0 g |
| Triacetin | 4,2 g |
| Wasser | 200,0 g |

Das Verhältnis Polyvinylacetat zu säureunlöslichem Polymer beträgt 85:15.

### Herstellung Coatingsuspension 1

2,3 g Triacetin und 1,7 g Polyvinylpyrrolidon K 90 wurden unter Rühren in 26,0 g Wasser gegeben und mit 50,0 g Kollicoat MAE 30 DP unter Rühren gemischt.

### Herstellung Coatingsuspension 2

4,2 g Triacetin wurden in 200,0 g Wasser gelöst und hierin 10,0 g Talkum unter Rühren dispergiert. Diese Suspension wurde anschließend langsam unter Rühren zu 283,3 g Kollicoat SR 30 D gegeben.

Die Zulufttemperatur betrug 65°C bei der Coatingsuspension 1 und 60°C bei der Coatingsuspension 2; die Sprührate lag bei 12 g/min. Das Coatinggewicht betrug 24 % der Verapamilpellets.

Die Freisetzung des Wirkstoffes wurde in 0,1 N HCl (-◆-) und in Phosphatpuffer (-■-) pH 7;4 bestimmt. Verapamil weist eine deutlich pH-abhängige Löslichkeit auf. Im Sauren beträgt sie 7,1 %, aber im Neutralbereich bzw. Alkalischen nur 0,2 %.

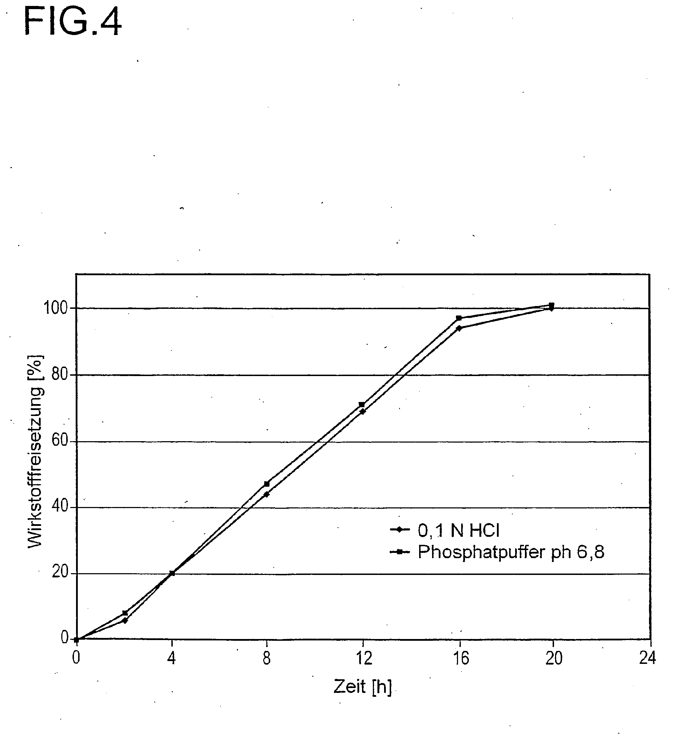

Die Freisetzungskurven sind in Abb. 4 dargestellt.

Die Freisetzung war in 0,1 N HCl und in Phosphatpuffer pH 7,4 gleich schnell.

### Vergleichsbeispiel 3

In der Rezeptur von Beispiel 3 wurde Ethylacrylat-Methacrylsäure-Copolymer weggelassen. Der Coatingprozess wurde analog durchgeführt.

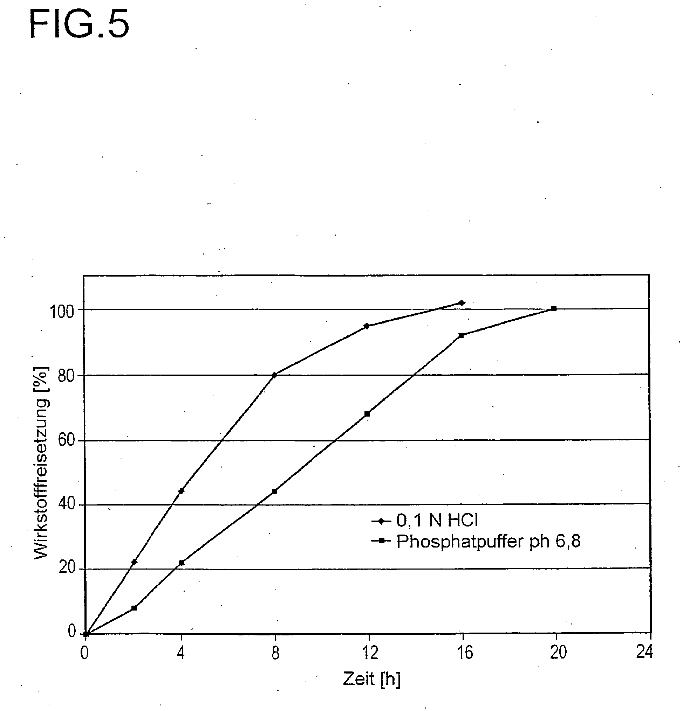

Die Freisetzung war in 0,1 N HCl (-◆-) schneller als in Phosphatpuffer (-■-) pH 7,4. Die Freisetzungskurven sind in Abb. 5 dargestellt.

### Beispiel 4

600 g Verapamilpellets (bestehend aus 10 % Verapamil-HCl, 0,5 % Hydroxypropylmethylcellulose 5 mPas, 0,05 % Polyethylenglycol 4000, 89,45 % Saccharose) mit einer Korngröße 0,8 bis 1,5 mm hergestellt nach dem Drug Layering-Verfahren wurden in einem Huettlin Kugelcoater HKC 5 mit folgenden Sprühsuspensionen gleichzeitig gecoatet:

### Sprühsuspension 1

| | |
|---|---|
| Polyvinylacetat-Dispersion 30 % (Kollicoat SR 30 D) | 352,0 g |
| Triethylcitrat | 10,6 g |
| Wasser | 327,4 g |

### Sprühsuspension 2

| | |
|---|---|
| Methacrylsäure-Ethylacrylat /1:1 Dispersion 30 % (Kollicoat MAE 30 DP) | 48,0 g |
| Triethylcitrat | 1,4 g |
| Wasser | 640,6 g |

Das Verhältnis Polyvinylacetat zu säureunlöslichem Polymer beträgt 88 : 13.

### Herstellung der Sprühsuspensionen:

Triethylcitrat wurde in der angegebenen Wassermenge gelöst und diese Lösung in die Kollicoat-Dispersion eingerührt.

Beide Dispersionen wurden parallel über getrennte Sprühdüsen auf die wirbelnden Verapamilpellets gesprüht.

Die Zulufttemperatur betrug 46°C und die Sprührate 6 g/min. Das Filmbildnergewicht, ausgedrückt als Summe von Polyvinylacetat und Methacrylsäure-Ethylacrylat Copolymer, betrug 20 % der Verapamilpellets.

Die Freisetzung des Wirkstoffes wurde in 0,1 N HCl und in Phosphatpuffer pH 6,8 bestimmt. Verapamil weist eine deutlich pH-abhängige Löslichkeit auf. Im Sauren beträgt sie 7,1 %, aber im Neutralbereich bzw. Alkalischen nur 0,2 %.

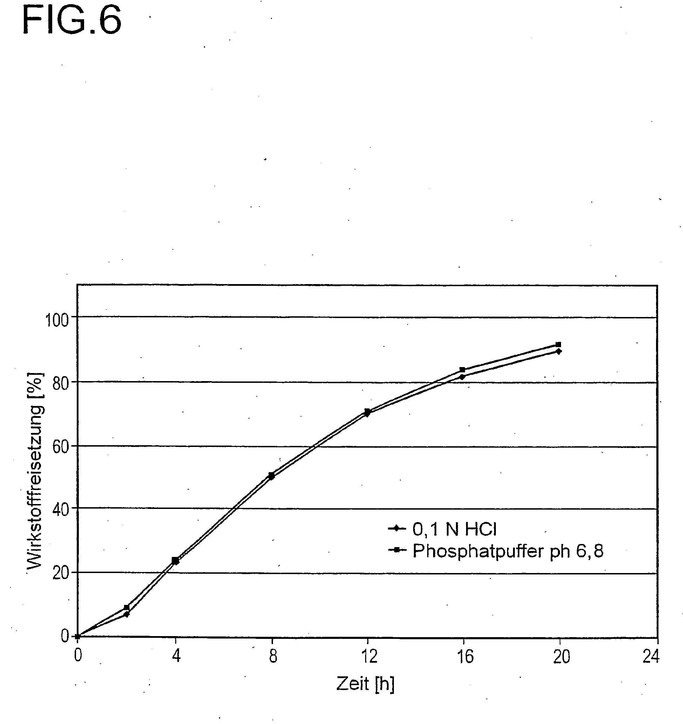

Die Freisetzung war in 0,1 N HCl (-◆-) und in Phosphatpuffer pH 6,8 (-■-) gleich schnell. Die Freisetzungskurven sind in Abb. 6 dargestellt.

### Beispiel 5

0,5 kg Propranolol-HCl Pellets (20 % Propranolol-HCl, 51,7 % mikrokristalline Cellulose, 25,8 % Lactose, 2,5 % Vinylpyrrolidon-Vinylacatat-(6:4)-Copolymer (Kollidon VA 64) mit einer Korngröße 0,4 bis 1,5 mm hergestellt durch Feuchtextrusion und anschließende Sphäronisation wurden in einem Wirbelschichtcoater Aeromatic Stream 1 mit folgender Coatingrezeptur gecoatet:

| | |
|---|---|
| Polyvinylacetat-Dispersion 30 % (Kollicoat SR 30 D) | 150,0 g |
| Ethylcellulose-Dispersion 30 % (Aquacoat ECD) | 100,0 g |
| Triacetin | 12,5 g |
| Talkum | 20,0 g |
| Vinylpyrrolidon-Vinylacetat- (6:4) -Copolymer | 2,5 g |
| Simethicon | 0,5 g |
| Wasser | 150,0 g |

Das Verhältnis Polyvinylacetat zu lipophilem Polymer betrug 3:2.

Zur Herstellung der Coatingrezeptur wurden Kollidon VA 64, Triacetin, Simethicon und Talkum in 150,0 g Wasser eingerührt und dispergiert. Diese Zubereitung wurde mit Kollicoat SR 30 D gemischt und anschließend mit Aquacoat ECD versetzt.

Die Coatingdispersion wurde bei einer Zulufttemperatur von 62°C und einer Sprührate von 12 g/min mittels Wurstereinsatz auf die wirbelnden Pellets appliziert. Das Coatinggewicht betrug 22 % der Propranololpellets.

Die Freisetzung des Wirkstoffes wurde 2 h in 0,1 N HCl und anschließend in Phosphatpuffer pH 6,8 bestimmt. Die gecoateten Pellets wurden mechanisch belastet (12 min Friabilatortest, entsprechend 300 Umdrehungen, Fallhöhe 15,5 cm) und wiederum die Freisetzung bestimmt.

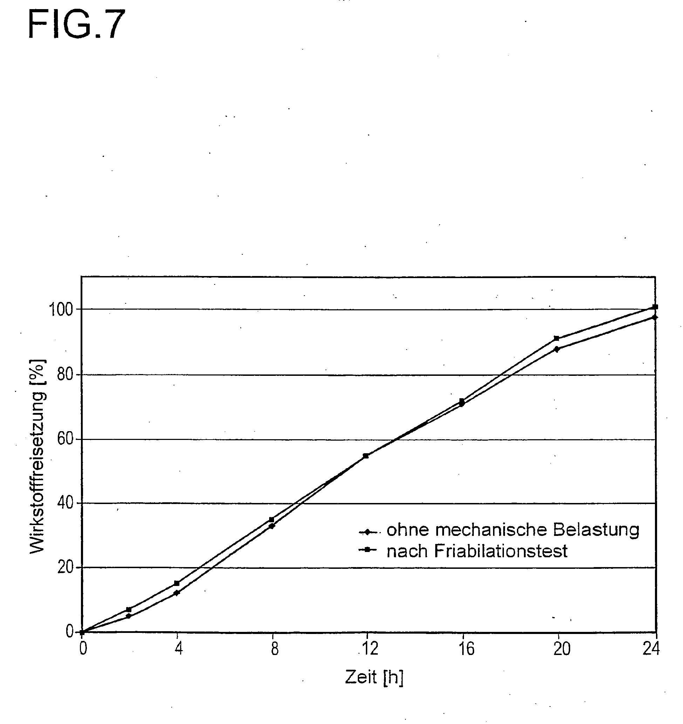

Eine mechanische Belastung hatte keine Auswirkungen auf die Freisetzung. Die Freisetzungskurven sind in Abb 7 dargestellt (-◆- ohne mechanische Belastung; **-■-** nach Friabilationstest).

### Vergleichsbeispiel 5

In der Rezeptur von Beispiel 5 wurde Polyvinylacetat weggelassen und durch weiteres Aquacoat ersetzt. Der Coatingprozess wurde analog durchgeführt.

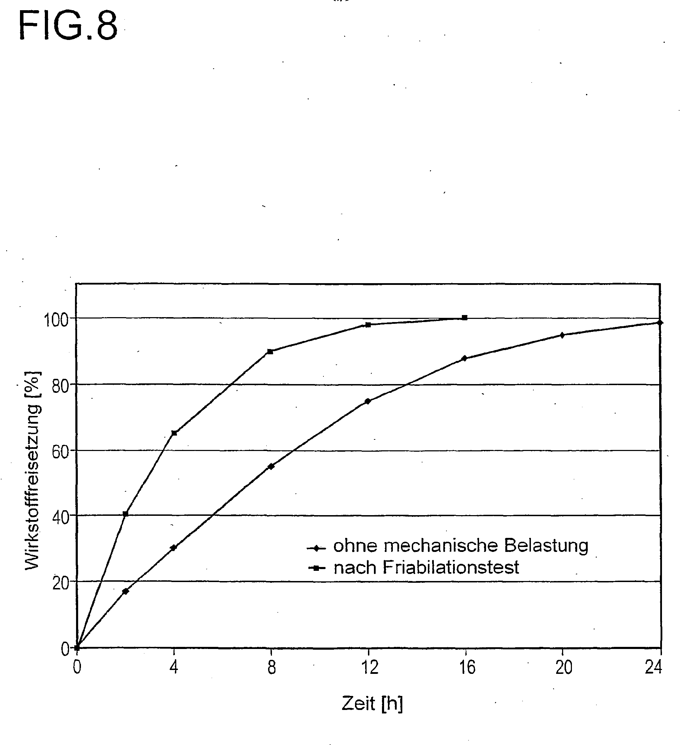

Die Freisetzung nach mechanischer Belastung war wesentlich schneller. Die Freisetzungskurven sind in Abb. 8 dargestellt (-◆- ohne mechanische Belastung; **-■-** nach Friabilationstest).

Die Pellets aus Beispiel 5 und dem Vergleichsbeispiel 5 wurden darüber hinaus nach folgender Rezeptur zu Tabletten mit einem Durchmesser von 10 mm und einem Gewicht von 400 mg verpresst:

| | |
|---|---|
| Gecoatete Propranololpellets | 250,0 g |
| Mikrokristalline Cellulose | 250,0 g |
| Magnesiumstearat | 2,5 g |

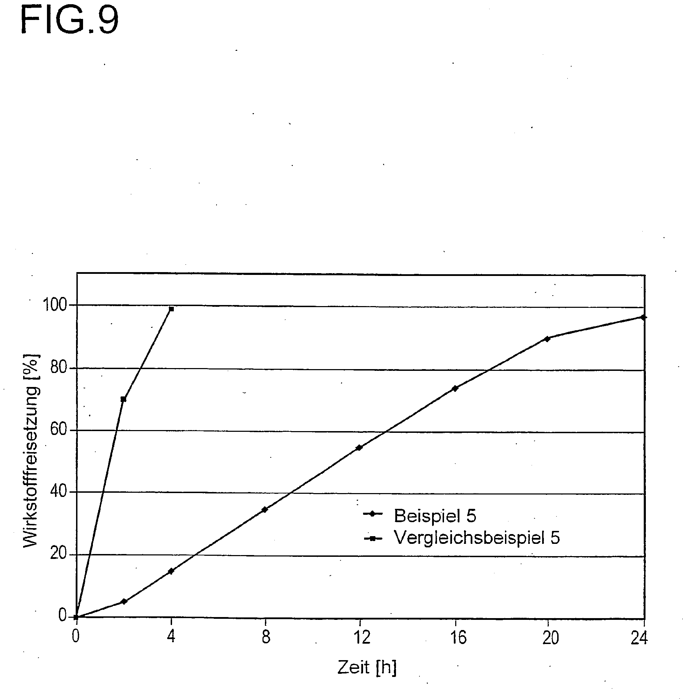

Von den Tabletten wurde ebenfalls die Freisetzung bestimmt. Die Freisetzungskurven sind in Abb. 9 dargestellt (-◆- verpresste Pellets gemäß Beispiel 5; -■- verpresste Pellets gemäß Vergleichsbeispiel 5).

Die mit Polyvinylacetat/Ethylcellulose gecoateten Pellets weisen auch nach Verpressung eine langsame Freisetzung auf, wohingegen die nur mit Ethylcellulose gecoateten eine deutlich beschleunigte Freisetzung zeigen.

In der nachfolgenden Tabelle ist die Wirkstofffreisetzung gemäß den Abb. 1 bis 9 in [%] angegeben.

**Tabelle: Wirkstofffreisetzung in [%]**

| Zeit | künstlicher Magensaft (0,1 N HCl) | künstlicher Darmsaft | Abb. Nr. |
|---|---|---|---|
| [h] | | (Phosphatpuffer pH 6,8) | |
| 0 | 0 | 0 | |
| 2 | 5 | 7 | |
| 4 | 10 | 13 | |
| 8 | 30 | 33 | 1 |
| 12 | 50 | 51 | |
| 16 | 70 | 70 | |
| 20 | 97 | 95 | |
| 24 | 100 | 98 | |
| 0 | 0 | 0 | |
| 2 | 8 | 17 | |
| 4 | 15 | 40 | |
| 8 | 35 | 80 | 2 |
| 12 | 57 | 100 | |
| 16 | 75 | - | |
| 20 | 95 | - | |
| 24 | 100 | - | |
| 0 | 0 | 0 | |
| 2 | 6 | 4 | |
| 4 | 18 | 20 | |
| 8 | 52 | 54 | 3 |
| 12 | 73 | 75 | |
| 16 | 84 | 86 | |
| 20 | 90 | 92 | |
| 24 | 100 | 100 | |
| 0 | 0 | 0 | |
| 2 | 6 | 8 | |
| 4 | 20 | 20 | |
| 8 | 44 | 47 | 4 |
| 12 | 69 | 71 | |
| 16 | 94 | 97 | |
| 20 | 100 | 101 | |
| 24 | - | - | |
| 0 | 0 | 0 | |
| 2 | 22 | 8 | |
| 4 | 44 | 22 | |
| 8 | 80 | 44 | 5 |
| 12 | 95 | 68 | |
| 16 | 102 | 92 | |
| 20 | - | 100 | |
| 24 | - | - | |
| 0 | 0 | 0 | |
| 2 | 7 | 9 | |
| 4 | 23 | 24 | 6 |
| 8 | 50 | 51 | |
| 12 | 70 | 71 | |

## Patentansprüche

1. Mit einem Filmüberzug versehene wirkstoffhaltige Darreichungsform mit kontrollierter, pH-unabhängiger Freisetzung, enthaltend als Wirkstoffe Arzneistoffe, Tierarzneistoffe, Vitamine, Carotinoide, Nutraceuticals, Nahrungsergänzungsmittel oder Zusatzstoffe, Mineralstoffe, Spurenelemente, wobei der Filmüberzug eine Schichtdicke zwischen 20 und 200 µm aufweist, und
(A) 10 - 99 Gew.-% Polyvinylacetat,
(B) 1 - 50 Gew.-% mindestens eines Polymers ausgewählt aus der Gruppe, bestehend aus lipophilen wasserunlöslichen Polymeren, säureunlöslichen Polymeren und alkaliunlöslichen Polymeren, und
(C) 0 - 50 Gew.-% weitere pharmazeutisch akzeptable Hilfsmittel
enthält, die Summe der Komponenten (A), (B) und (C) 100 Gew.-% beträgt und das Verhältnis von (A) zu (B) 70:30 bis 99:1 beträgt.

2. Darreichungsform nach Anspruch 1, wobei die lipophilen wasserunlöslichen Polymere ausgewählt sind aus der Gruppe bestehend aus Alkylcellulosen, Acrylat-Methacrylat-Copolymeren, Vinylacetat-Methacrylat und -Acrylat-Copolymeren.

3. Darreichungsform nach Anspruch 1 oder 2, wobei die säureunlöslichen Polymere ausgewählt sind aus der Gruppe bestehend aus Acrylat-Methacrylsäure-Copolymeren, Carboxyalkylcellulosen, Celluloseacetatphthalaten, Celluloseacetatsuccinaten, Celluloseacetattrimellitaten, Hydroxyalkylcellulosephthalaten, Hydroxyalkylcelluloseacetatsuccinaten, Vinylacetatphthalaten und Vinylacetatsuccinaten.

4. Darreichungsform nach einem der Ansprüche 1 bis 3, wobei die alkaliunlöslichen Polymere ausgewählt sind aus der Gruppe bestehend aus basischen Acrylat-Methacrylat-Copolymeren und basischen natürlichen Polysacchariden.

5. Darreichungsform nach einem der Ansprüche 1 bis 4, wobei der Filmüberzug 20 bis 80 Gew.-% Polymere (A) und 5 bis 40 Gew.-% Polymere (B) enthält.

6. Darreichungsform nach einem der Ansprüche 1 bis 5, wobei als lipophiles wasserunlösliches Polymer Ethylcellulose, Ethylacrylat-Methylmethacrylat-Copolymer und Ethylacrylat-Methylmethacrylat-Trimethylammoniummethylmethacrylatchlorid-Terpolymer oder deren Gemische eingesetzt werden.

7. Darreichungsform nach einem der Ansprüche 1 bis 6, wobei als säureunlösliches Polymer Ethylacrylat-Methacrylsäure-Copolymer, Methylmethacrylat-Methacrylsäure-Copolymer, Methylmethacrylat-Methylacrylat-Methacrylsäure-Copolymer, Carboxymethylcellulose, Celluloseacetatphthalat, Hydroxypropylmethylcellulosephthalat, Hydroxypropylmethylcelluloseacetatphthalat, Hydroxypropylmethylcelluloseacetatsuccinat, Polyvinylacetatphthalat, Schellack oder deren Gemische eingesetzt werden.

8. Darreichungsform nach einem der Ansprüche 1 bis 7, wobei als alkaliunlösliches Polymer Dimethylaminoethylmethacrylat-Methylmethacrylat-Butylmethacrylat-Terpolymer, Chitosan oder deren Gemische eingesetzt werden.

9. Darreichungsformen nach einem der Ansprüche 1 bis 8, wobei das Polyvinylacetat ein Molekulargewicht von 10000 bis 2000000 vorzugsweise von 100000 bis 1000000 aufweist.

10. Darreichungsformen nach einem der Ansprüche 1 bis 9, wobei Polyvinylacetat und gegebenenfalls die lipophilen, wasserunlöslichen oder säureunlöslichen oder alkaliunlöslichen Polymere zur Herstellung des Filmüberzugs in Form einer wässrigen Dispersion eingesetzt werden.

11. Darreichungsformen nach einem der Ansprüche 1 bis 10, wobei alle Filmüberzugsbestandteile vor dem Herstellen des Filmüberzugs gemischt werden, gegebenenfalls unter Zusatz eines oberflächenaktiven Stoffes.

12. Darreichungsformen nach einem der Ansprüche 1 bis 11, wobei Polyvinylacetat und die lipophilen, wasserunlöslichen oder säureunlöslichen oder alkaliunlöslichen Polymere in zwei oder mehreren Schichten appliziert werden, die sich in ihrem Verhältnis Polyvinylacetat zu lipophilen, wasserunlöslichen oder säureunlöslichen oder alkaliunlöslichen Polymeren unterscheiden.

13. Darreichungsformen nach einem der Ansprüche 1 bis 12, wobei polyvinylacetat und die lipophilen, wasserunlöslichen oder säureunlöslichen oder alkaliunlöslichen Polymere in zwei oder mehreren Schichten appliziert werden, wobei die eine Polyvinylacetat und die andere das lipophile wasserunlösliche oder säureunlösliche oder alkaliunlösliche Polymer enthält.

14. Darreichungsformen nach einem der Ansprüche 1 bis 13, wobei der Filmüberzug zusätzlich hydrophile, wasserlösliche Polymere oder niedermolekulare, wasserlösliche Stoffe enthält.

15. Darreichungsformen nach einem der Ansprüche 1 bis 14, in Form von Pellets, Granulaten, Kristallen, Extrudaten, Tabletten oder Drug Delivery Systemen.

16. Tabletten, hergestellt durch Verpressung der überzogenen Darreichungsformen gemäß den Ansprüchen 1 bis 15, zusammen mit üblichen pharmazeutischen Hilfsstoffen.

17. Verfahren zur Herstellung der erfindungsgemäßen Darreichungsformen nach einem der Ansprüche 1 bis 15, **dadurch gekennzeichnet, dass** eine wässrige Filmüberzugszubereitung auf sich bewegende Formlinge aufgesprüht wird und simultan durch Zufuhr warmer Luft abgetrocknet wird, wobei Polyvinylacetat und die lipophilen wasserunlöslichen oder säureunlöslichen oder alkaliunlöslichen Polymere in der Überzugsvorrichtung über getrennte Sprühdüsen appliziert werden.

## Claims

1. An active ingredient-containing dosage form which is provided with a film coating and has controlled, pH-independent release, comprising medicinal substances, veterinary medicinal substances, vitamins, carotenoids, nutraceuticals, food supplements or additives, minerals, trace elements or active ingredients, where the film coating has a thickness of between 20 and 200 µm and comprises
(A) 10 - 99% by weight of polyvinyl acetate,
(B) 1 - 50% by weight of at least one polymer selected from the group consisting of lipophilic water-insoluble polymers, acid-insoluble polymers and alkali-insoluble polymers, and
(C) 0 - 50% by weight of other pharmaceutically acceptable aids,
and the total of components (A), (B) and (C) is 100% by weight and where the ratio of (A) to (B) is from 70:30 to 99:1.

2. The dosage form according to claim 1, where the lipophilic water-insoluble polymers are selected from the group consisting of alkylcelluloses, acrylate/methacrylate copolymers, vinyl acetate/methacrylate and vinyl acetate/acrylate copolymers.

3. The dosage form according to claim 1 or 2, where the acid-insoluble polymers are selected from the group consisting of acrylate/methacrylic acid copolymers, carboxyalkylcelluloses, cellulose acetate phthalates, cellulose acetate succinates, cellulose acetate trimellitates, hydroxyalkylcellulose phthalates, hydroxyalkylcellulose acetate succinates, vinyl acetate phthalates and vinyl acetate succinates.

4. The dosage form according to any of claims 1 to 3, where the alkali-insoluble polymers are selected from the group consisting of basic acrylate/methacrylate copolymers and basic natural polysaccharides.

5. The dosage form according to any of claims 1 to 4, where the film coating comprises 20 to 80% by weight of polymers (A) and 5 to 40% by weight of polymers (B).

6. The dosage form according to any of claims 1 to 5, where ethylcellulose, ethyl acrylate/methyl methacrylate copolymer and ethyl acrylate/methyl methacrylate/trimethylammoniumethyl methacrylate chloride terpolymer or mixtures thereof are employed as lipophilic water-insoluble polymer.

7. The dosage form according to any of claims 1 to 6, where ethyl acrylate/methacrylic acid copolymer, methyl methacrylate/methacrylic acid copolymer, methyl methacrylate/methyl acrylate/methacrylic acid copolymer, carboxymethylcellulose, cellulose acetate phthalate, hydroxypropylmethylcellulose phthalate, hydroxypropylmethylcellulose acetate phthalate, hydroxypropylmethylcellulose acetate succinate, polyvinyl acetate phthalate, shellac and mixtures thereof are employed as acid-insoluble polymer.

8. The dosage form according to any of claims 1 to 7, where dimethylaminoethyl methacrylate/methyl methacrylate/butyl methacrylate terpolymer, chitosan or mixtures thereof are employed as alkali-insoluble polymer.

9. The dosage form according to any of claims 1 to 8, where the polyvinyl acetate has a molecular weight of from 10 000 to 2 000 000, preferably from 100 000 to 1 000 000.

10. The dosage form according to any of claims 1 to 9, where polyvinyl acetate and, where appropriate, the lipophilic, water-insoluble or acid-insoluble or alkali-insoluble polymers are employed in the form of an aqueous dispersion for producing the film coating.

11. The dosage form according to any of claims 1 to 10, where all the film coating ingredients are mixed before production of the film coating, where appropriate with the addition of a surface-active substance.

12. The dosage form according to any of claims 1 to 11, where polyvinyl acetate and the lipophilic, water-insoluble or acid-insoluble or alkali-insoluble polymers are applied in two or more layers which differ in their ratio of polyvinyl acetate to lipophilic, water-insoluble or acid-insoluble or alkali-insoluble polymers.

13. The dosage form according to any of claims 1 to 12, where polyvinyl acetate and the lipophilic, water-insoluble or acid-insoluble or alkali-insoluble polymers are applied in two or more layers, one comprising polyvinyl acetate and the other comprising the lipophilic water-insoluble or acid-insoluble or alkali-insoluble polymer.

14. The dosage form according to any of claims 1 to 13, where the film coating additionally comprises hydrophilic, water-soluble polymers or low molecular weight, water-soluble substances.

15. The dosage form according to any of claims 1 to 14, in the form of pellets, granules, crystals, extrudates, tablets or drug delivery systems.

16. A tablet produced by compression of the coated dosage form according to claims 1 to 15 together with conventional pharmaceutical excipients.

17. A process for preparation of the dosage forms of the invention according to any of claims 1 to 21, which comprises an aqueous film coating preparation being sprayed onto shaped articles in motion and simultaneously being dried by input of hot air, where polyvinyl acetate and the lipophilic water-insoluble or acid-onsoluble or alkali-insoluble polymers are applied throuh separate spray nozzles in the coating device.

## Revendications

1. Forme de préparation ayant une teneur en substance active et étant pourvue d'un film de revêtement, qui présente une libération contrôlée, indépendante du pH, et contient, comme substances actives, des médicaments, des médicaments vétérinaires, des vitamines, des caroténoïdes, des nutraceutiques, des compléments ou additifs alimentaires, des substance minérales, des oligo-éléments, le film de revêtement présentant une épaisseur de couche comprise entre 20 et 200 µm, et contenant
(A) 10-99 % en poids d'acétate de polyvinyle,
(B) 1- 50 % en poids d'au moins un polymère choisi parmi le groupe constitué des polymères insolubles dans l'eau lipophiles, des polymères insolubles dans un acide et des polymères insolubles dans un alcali, et
(C)0-50 % en poids d'autres adjuvants pharmaceutiquement acceptables,
la somme des composants (A), (B) et (C) étant de 100 % en poids et le rapport entre (A) et (B) étant de 70/30 à 99/1.

2. Forme de préparation suivant la revendication 1, dans laquelle les polymères insolubles dans l'eau lipophiles sont choisis parmi le groupe constitué des alkylcelluloses, des copolymères d'acrylate-méthacrylate, des copolymère d'acétate de vinyle-méthacrylate et d'acétate de vinyle-acrylate.

3. Forme de préparation suivant la revendication 1 ou 2, dans laquelle les polymères insolubles dans un acide sont choisis parmi le groupe constitué des copolymères d'acrylate-acide méthacrylique, des carboxyalkylcelluloses, des acétophtalates de cellulose, des acétosuccinates de cellulose, des acétotrimellitates de cellulose, des phtalates d'hydroxyalkylcellulose, des acétosuccinates d'hydroxyalkylcellulose, des acétophtalates de vinyle et des acétosuccinates de vinyle.

4. Forme de préparation suivant l'une des revendications 1 à 3, dans laquelle les polymères insolubles dans un alcali sont choisis parmi le groupe constitué des copolymères d'acrylate-méthacrylate basiques et des polysaccharides naturels basiques.

5. Forme de préparation suivant l'une des revendications 1 à 4, dans laquelle le film de revêtement contient 20 à 80 % en poids de polymères (A) et 5 à 40 % en poids de polymère (B).

6. Forme de préparation suivant l'une des revendications 1 à 5, dans laquelle, comme polymère insoluble dans l'eau lipophile, on met en oeuvre de l'éthylcellulose, un copolymère acrylate d'éthyle-méthacrylate de méthyle et un terpolymère d'acrylate d'éthyle-méthacrylate de méthyle-chlorure de méthylméthacrylate de triméthylammonium ou leurs mélanges.

7. Forme de préparation suivant l'une des revendications 1 à 6, dans laquelle, comme polymère insoluble dans un acide, on utilise un copolymère d'acrylate d'éthyle-acide méthacrylique, un copolymère de méthacrylate de méthyle-acide méthacrylique, un copolymère de méthacrylate de méthyle-acrylate de méthyle-acide méthacrylique, de la carboxyméthylcellulose, de l'acétophtalate de cellulose, du phtalate d'hydroxypropylméthylcellulose, de l'acétophtalate d'hydroxypropylméthylcellulose, de l'acétosuccinate d'hydroxypropylméthylcellulose, de l'acétophtalate de polyvinyle, de la gomme-laque ou leurs mélanges.

8. Forme de préparation suivant l'une des revendications 1 à 7, dans laquelle, comme polymère insoluble dans un alcali, on met en oeuvre un terpolymère de méthacrylate de diméthylaminoéthyle-méthacrylate de méthyle-méthacrylate de butyle, du chitosan ou leurs mélanges.

9. Formes de préparation suivant l'une des revendications 1 à 8, dans lesquelles l'acétate de polyvinyle présente un poids moléculaire de 10 000 à 2 000 000, de préférence de 100 000 à 1 000 000.

10. Formes de préparation suivant l'une des revendications 1 à 9, dans lesquelles on met en oeuvre de l'acétate de polyvinyle et éventuellement les polymères insolubles dans l'eau lipophiles ou insolubles dans un acide ou insolubles dans un alcali pour la préparation du film de revêtement sous la forme d'une dispersion aqueuse.

11. Formes de préparation suivant l'une des revendications 1 à 10, dans lesquelles on mélange tous les éléments du film de revêtement avant la préparation du film de revêtement, éventuellement avec addition d'une substance tensioactive.

12. Formes de préparation suivant l'une des revendications 1 à 11, dans lesquelles l'acétate de polyvinyle et les polymères insolubles dans l'eau lipophiles ou insolubles dans un acide ou insolubles dans un alcali sont appliqués en deux ou plusieurs couches qui se différencient dans leur proportion d'acétate de polyvinyle par rapport aux polymères insolubles dans l'eau lipophiles ou insolubles dans un acide ou insolubles dans un alcali.

13. Formes de préparation suivant l'une des revendications 1 à 12, dans lesquelles l'acétate de polyvinyle et les polymères insolubles dans l'eau lipophiles ou insolubles dans un acide ou insolubles dans un alcali sont appliqués en deux ou plusieurs couches, l'une contenant de l'acétate de polyvinyle et les autres le polymère insoluble dans l'eau lipophile ou insoluble dans un acide ou insoluble dans un alcali.

14. Formes de préparation suivant l'une des revendications 1 à 13, dans lesquelles le film de revêtement contient en supplément des polymères solubles dans l'eau hydrophiles ou des substances solubles dans l'eau de faible poids moléculaire.

15. Formes de préparation suivant l'une des revendications 1 à 14, sous la forme de pastilles, de granules, de cristaux, d'extrudés, de comprimés ou de systèmes de libération de médicaments.

16. Comprimés, préparés par compression des formes de préparation revêtues suivant les revendications 1 à 15, conjointement à des adjuvants pharmaceutiques courants.

17. Procédé de préparation des formes de préparation suivant l'invention selon une des revendications 1 à 15, **caractérisé en ce qu'**une composition aqueuse de film de revêtement est pulvérisée sur des agglomérés en mouvement et est simultanément séchée par un apport d'air chaud, l'acétate de polyvinyle et les polymères insolubles dans l'eau lipophiles ou insolubles dans un acide ou insolubles dans un alcali étant appliqués dans le dispositif de revêtement par l'intermédiaire de tuyères de pulvérisation séparées.
